(19) Europäisches Patentamt
European Patent Office
Office européen des brevets

(11) **EP 4 354 119 A1**

(12) **EUROPEAN PATENT APPLICATION**
published in accordance with Art. 153(4) EPC

(43) Date of publication:
**17.04.2024 Bulletin 2024/16**

(21) Application number: **22820128.1**

(22) Date of filing: **02.06.2022**

(51) International Patent Classification (IPC):
**G01N 21/64** (2006.01)      **G01N 33/543** (2006.01)

(52) Cooperative Patent Classification (CPC):
**C09K 11/06; G01N 21/64; G01N 33/543**

(86) International application number:
**PCT/JP2022/022413**

(87) International publication number:
**WO 2022/259946 (15.12.2022 Gazette 2022/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.06.2021 JP 2021096211**

(71) Applicants:
• **CANON KABUSHIKI KAISHA**
  **Tokyo 146-8501 (JP)**
• **Canon Medical Systems Corporation**
  **Tochigi 324-0036 (JP)**

(72) Inventors:
• **KAKEGAWA Norishige**
  **Tokyo 146-8501 (JP)**
• **MASUMURA Takahiro**
  **Tokyo 146-8501 (JP)**
• **SAKAKIBARA Teigo**
  **Tokyo 146-8501 (JP)**
• **NAKAJIMA Ikuo**
  **Tokyo 146-8501 (JP)**
• **YAMAUCHI Fumio**
  **Tokyo 146-8501 (JP)**
• **KANAZAKI Kengo**
  **Tokyo 146-8501 (JP)**

(74) Representative: **TBK**
**Bavariaring 4-6**
**80336 München (DE)**

(54) **METHOD FOR DETECTING AND MEASURING TARGET SUBSTANCE ON BASIS OF MEASUREMENT OF POLARIZATION ANISOTROPY, AND PARTICLES USED THEREFOR**

(57)      To provide a method and kit for measurement of a target substance with high detection sensitivity, provided is a method of measuring at least any one of the presence or absence, and a concentration, of a target substance in a sample liquid, the method including the steps of: (a) preparing a first particle that specifically binds to the target substance and contains a rare earth complex, and a second particle that has a larger average particle diameter than that of the first particle and specifically binds to the target substance; (b) mixing the sample liquid, the first particle, and the second particle to provide a mixed liquid; and (c) determining at least any one of the presence or absence, and the concentration, of the target substance from polarization anisotropy of the mixed liquid obtained in the step (b).

FIG. 1

EP 4 354 119 A1

**Description**

[Technical Field]

The present invention relates to a method of detecting and measuring a target substance in a sample to be tested, and to particles to be used for the method.

[Background Art]

In the fields of medicine and clinical tests, high-sensitivity detection of a trace amount of a biological component from, for example, blood or a collected part of an organ is required for investigating the cause of a disease. Among detection techniques for biological components, immunoassays are widely utilized. As one of the immunoassays, there is known a latex agglutination method utilizing an antigen-antibody reaction. The "latex agglutination method" is a method that is aimed at detecting an antigen in a liquid sample such as a biological sample, and that includes mixing latex having supported thereon, for example, an antibody that specifically binds to the antigen with the liquid sample, and measuring the degree of agglutination of the latex, to thereby detect or quantify the antigen.

In the latex agglutination method, the antigen is captured by the antibody bound to the latex, and a plurality of particles of the latex are crosslinked via the captured antigen, with the result that the agglutination occurs. That is, the amount of the antigen in a liquid sample such as a biological sample can be quantified by evaluating the degree of the agglutination of the latex. The degree of the agglutination can be quantified by evaluating a change in amount of light transmitted through or scattered by the liquid sample.

The latex agglutination method can quantitatively evaluate the antigen in a simple and rapid manner, but has involved a problem in that the antigen cannot be detected when its amount in the liquid sample such as the biological sample is small.

In order to improve detection sensitivity, it is conceivable to replace a system for scattering transmitted light with a method involving detecting a luminescence characteristic with higher sensitivity. Specifically, there has been proposed, for example, a specimen test method utilizing a fluorescence depolarization method (Patent Literatures 1 to 3).

In Patent Literature 1, it is proposed that an apparatus for the fluorescence depolarization method be improved to be clinically used. The fluorescence depolarization method does not require preliminary separation between a measurement substance and an unreacted luminescent substance, that is, a washing step called bound/free (B/F) separation, which is required in a general fluorescence measurement method. Accordingly, a simple specimen test can be performed as in the latex agglutination method.

Further, it is conceived that measurement can be performed by the same test system as in the latex agglutination method by merely mixing a luminescent substance that specifically reacts with the measurement substance in a measurement process. However, use of a single molecule such as fluorescein as a luminescent material, which is proposed in Patent Literature 1, is applicable only to a drug, a low-molecular-weight antigen, and the like in principle.

In Patent Literature 2, in order to enable detection of a high-molecular-weight substance such as a protein, which is difficult in Patent Literature 1, it is proposed to use a material obtained by adsorbing a dye having a long luminescence lifetime onto latex particles. In Patent Literature 2, it is proposed that measurement of a high-molecular-weight substance be performed by balancing a reduction in rotational Brownian motion of the substance in a liquid due to an increase in particle diameter and the length of luminescence lifetime based on the principle of the fluorescence depolarization method. However, bovine serum albumin (BSA), which is a biomolecule, is supported on surfaces of the particles for the purpose of suppressing nonspecific adsorption, and hence a lot-to-lot variation may occur owing to a broadened particle size distribution and BSA, which is a protein. As a result, it can hardly be said that antigen detection with extremely high sensitivity has been achieved.

In Patent Literature 3, there is proposed a method of performing measurement by causing a fluorescent label and a target antigen to be competitively adsorbed onto a large particle through an antigen-antibody reaction. However, in this method, one antibody reacts with one fluorescent label, and hence it is difficult to grasp a change in degree of polarization with high sensitivity.

[Citation List]

[Patent Literature]

PTL 1: Japanese Patent Publication No. H03-52575
PTL 2: Japanese Patent No. 2893772

PTL 3: Japanese Patent Application Laid-Open No. H03-103765

[Summary of Invention]

[Technical Problem]

The present invention has been made in view of such background art, and an object of the present invention is to provide a high-sensitivity measurement method and test kit based on a change in polarization anisotropy. In particular, there is provided a technique for more sensitively grasping a reaction by increasing the change in polarization anisotropy.

[Solution to Problem]

The present invention provides a method of determining at least any one of the presence or absence, and a concentration, of a target substance in a sample liquid, the method including the following steps:

(a) preparing a first particle that specifically binds to the target substance and contains a rare earth complex, and a second particle that has a larger average particle diameter than that of the first particle and specifically binds to the target substance;
(b) mixing the sample liquid, the first particle, and the second particle to provide a mixed liquid; and
(c) determining at least any one of the presence or absence, and the concentration, of the target substance from polarization anisotropy of the mixed liquid obtained in the step (b).

The present invention also provides a test kit including a first particle and a second particle, and a method of producing the same.

[Advantageous Effects of Invention]

According to the present invention, the aggregation of the particles in a liquid can be detected with high sensitivity through measurement using polarized light, and thus the target substance can be measured with high sensitivity. In particular, a reaction can be more sensitively grasped from a change in polarization anisotropy detected when the particles aggregate in the liquid.
In addition, particles each having a high suppressing effect on nonspecific adsorption without use of BSA or the like by forming a hydrophilic layer on the surface of the particle are proposed. Accordingly, the particles of the present invention are more advantageously used for measurement based on polarization anisotropy, enabling measurement with even higher sensitivity.

[Brief Description of Drawings]

[FIG. 1]

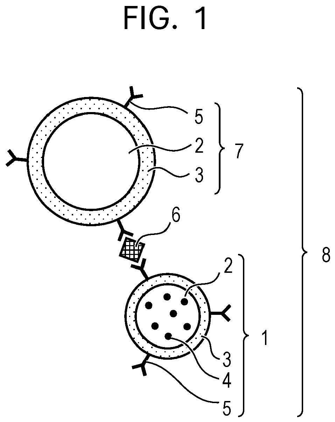

FIG. 1 is a view for illustrating a measurement method according to an embodiment of the present invention.

[Description of Embodiments]

Preferred embodiments of the present invention are described in detail below. However, the embodiments are not intended to limit the scope of the present invention.
The present invention provides, as one embodiment thereof, a method of determining at least any one of the presence or absence, and a concentration, of a target substance in a sample liquid, the method including the following steps:

(a) preparing a first particle that specifically binds to the target substance and contains a rare earth complex, and a second particle that has a larger average particle diameter than that of the first particle and specifically binds to the target substance;
(b) mixing the sample liquid, the first particle, and the second particle to provide a mixed liquid; and
(c) determining at least any one of the presence or absence, and the concentration, of the target substance from polarization anisotropy of the mixed liquid obtained in the step (b).

The polarization anisotropy is an index showing the tendency of luminescence emitted by an object irradiated with polarized light to keep a component in the orientation of the radiated polarized light. The polarization anisotropy is defined as described below. That is, the polarization anisotropy is a value showing a relationship between the luminescence intensity of a polarized light component of luminescence generated by exciting a luminescent substance through irradiation with polarized light, the component being parallel to the polarized light that is incident light, and the luminescence intensity of a polarized light component in a perpendicular direction thereto. More specifically, the polarization anisotropy is a value calculated from the luminescence intensity of a luminescence component having a vibration direction parallel to that of given polarized light, the luminescence intensity being determined when the luminescent substance is excited by the polarized light. Further, the polarization anisotropy is a value indicating the ratio of a difference between the luminescence intensity of a luminescence component having a vibration direction parallel to that of a first polarized light beam at the time of excitation by the first polarized light beam and the luminescence intensity of a luminescence component having a vibration direction orthogonal to that of the first polarized light beam at the time of excitation by the first polarized light beam to the sum of the luminescence intensities. The polarization anisotropy may be corrected with: a ratio between the luminescence intensity of a luminescence component having a vibration direction orthogonal to that of a second polarized light beam having a vibration direction orthogonal to that of the first polarized light beam at the time of excitation by the second polarized light beam and the luminescence intensity of a luminescence component having a vibration direction parallel to that of the second polarized light beam having a vibration direction orthogonal to that of the first polarized light beam at the time of excitation by the second polarized light beam; and other constants.

More specifically, the polarization anisotropy is represented by <r> in the following equation 1:

[Math. 1]

$$< r >= \frac{I_{VV} - G * I_{VH}}{I_{VV} + 2 * G * I_{VH}} \qquad G = \frac{I_{HV}}{I_{HH}} \qquad \text{(Equation 1)}$$

where

<r> represents the polarization anisotropy,

Ivv represents the luminescence intensity of a luminescence component having a vibration direction parallel to that of a first polarized light beam at the time of excitation by the first polarized light beam,

$I_{VH}$ represents the luminescence intensity of a luminescence component having a vibration direction orthogonal to that of the first polarized light beam at the time of excitation by the first polarized light beam,

$I_{HV}$ represents the luminescence intensity of a luminescence component having a vibration direction orthogonal to that of a second polarized light beam having a vibration direction orthogonal to that of the first polarized light beam at the time of excitation by the second polarized light beam,

$I_{HH}$ represents the luminescence intensity of a luminescence component having a vibration direction parallel to that of the second polarized light beam having a vibration direction orthogonal to that of the first polarized light beam at the time of excitation by the second polarized light beam, and

G represents a correction value.

An object is excited with the first polarized light beam by, for example, arranging a polarizer on the incidence side of light. When the intensity of luminescence is measured with a polarizer arranged on the detection side in a parallel direction to the polarizer on the incidence side, the luminescence intensity of the luminescence component having a vibration direction parallel to that of the first polarized light beam, that is, Ivv can be measured. Meanwhile, when the intensity of luminescence is measured with a polarizer set on the detection side in a direction orthogonal to the polarizer on the incidence side, the luminescence intensity of the luminescence component having a vibration direction orthogonal to that of the first polarized light beam, that is, $I_{VH}$ can be measured.

The object is excited by arranging a polarizer on the incidence side of light in a direction orthogonal to that at the time when the first polarized light beam was obtained, that is, with the second polarized light beam. When the intensity of luminescence is measured with a polarizer arranged on the detection side in a parallel direction to the polarizer on the incidence side, the luminescence intensity of the luminescence component having a vibration direction parallel to that of the second polarized light beam, that is, $I_{HV}$ can be measured. Meanwhile, when the intensity of luminescence is measured with a polarizer set on the detection side in a direction orthogonal to the polarizer on the incidence side, the luminescence intensity of the luminescence component having a vibration direction orthogonal to that of the second polarized light beam, that is, $I_{HH}$ can be measured.

In addition, the polarization anisotropy may be represented by <r'> in the following equation 2.

[Math. 2]

$$<r>' = \frac{I_{VV} - G * I_{VH}}{I_{VV} + G * I_{VH}}$$

$$G = \frac{I_{HV}}{I_{HH}}$$

(Equation 2)

Each symbol is the same as that in the equation (1).

With regard to conditions for the measurement of the polarization anisotropy, for example, it is preferred that the measurement be performed in a liquid having a temperature of from 0°C to 50°C, and the viscosity of the liquid be from 0.5 mPa·s to 50 mPa·s. When a luminescent reagent is particles each containing a rare earth complex, the measurement is preferably performed with the concentration of the luminescent reagent being set to from 0.001 mg/ml to 0.1 mg/ml, and an excitation wavelength is preferably from 500 nm to 700 nm.

In addition, the present invention provides, as one embodiment thereof, a test kit for determining at least any one of the presence or absence, and the concentration, of a target substance.

The test kit includes a first particle and a second particle, and the first particle specifically binds to the target substance and contains a rare earth complex. The second particle has a larger average particle diameter than that of the first particle, and specifically binds to the target substance.

The phrase "specifically binds to the target substance" refers to having a property of specifically interacting with the target substance, and particularly preferably refers to having an ability to specifically bind to the target substance. As a mechanism of the binding, there may be given an electrostatic interaction, a van der Waals interaction, an interaction by a hydrogen bond, and the like. The first particle and the second particle may be identical to or different from each other in terms of a site for binding to the target substance or the mechanism.

Any substance may serve as the target substance as long as the substance shows an interaction with some substance.

Examples of the target substance may include an antigen, an antibody, a low-molecular-weight compound, various receptors, an enzyme, a substrate, a nucleic acid, a cytokine, a hormone, a neurotransmitter, a transmitter, and a membrane protein. Examples of the antigen include an allergen, a bacterium, a virus, a cell, a cell membrane constituent, a cancer marker, various disease markers, an antibody, a blood-derived substance, a food-derived substance, a natural product-derived substance, and any low-molecular-weight compound. Examples of the nucleic acid include DNA, RNA, or cDNA derived from a bacterium, a virus, or a cell, a part or fragment thereof, a synthetic nucleic acid, a primer, and a probe. Examples of the low-molecular-weight compound include a cytokine, a hormone, a neurotransmitter, a transmitter, a membrane protein, and receptors therefor.

The first particle contains a rare earth complex, and has a luminescent property. That is, the first particle has a property of showing luminescence when irradiated with an excitation wavelength. The rare earth complex of the first particle is a rare earth complex having a luminescent property. Preferred examples thereof may include complexes of europium, terbium, neodymium, erbium, yttrium, lanthanum, cerium, samarium, gadolinium, dysprosium, thulium, ytterbium, and scandium. The luminescence of the rare earth complex has a long lifetime. The polarization anisotropy depends on a change in rotational motion of a luminescent material during a luminescence time, and hence the use of the rare earth complex having a long luminescence lifetime as the luminescent substance is preferred.

The first particle and the second particle form an aggregate via the target substance. It is desired that the first particle have relatively low polarization anisotropy before being mixed with the sample liquid, that is, the polarization anisotropy to be measured be low, and depolarization of fluorescence occur.

Meanwhile, when the particles aggregate together via the target substance, the polarization anisotropy becomes relatively high. That is, it is desired that part (or the entirety) of the depolarization of fluorescence be canceled.

The first particle and the second particle form an aggregate in a dispersion medium via the target substance. Aggregation between the first particles, and aggregation between the second particles may also occur. The polarization anisotropy that the aggregate shows in the dispersion medium is preferably increased as compared to the polarization anisotropy that the unaggregated first particle shows in the dispersion medium. The polarization anisotropy is preferably increased by 0.004 or more, more preferably increased by 0.01 or more, still more preferably increased by 0.02 or more.

The first particle shows a polarization anisotropy of preferably 0.2 or less, more preferably 0.15 or less, still more preferably 0.12 or less in the dispersion medium.

In addition, the aggregate shows a polarization anisotropy of preferably 0.05 or more, more preferably 0.09 or more, still more preferably 0.13 or more in the dispersion medium.

In addition, the first particle and the second particle that are used for the measurement preferably account for from 0.000001 mass% to 1 mass% in total in the dispersion medium, and their weight ratio preferably falls within the range of from 1:9 to 9:1.

In the measurement for determining the polarization anisotropy, polarized excitation light (the first polarized light beam) may be radiated by arranging a polarizer such as a polarizing filter on the incidence side of the light. When a polarizing filter is arranged in a direction orthogonal to the foregoing, the second polarized light beam having a vibration direction orthogonal to that of the first polarized light beam can be radiated. When a polarizer is arranged on the detection side in a parallel direction to the polarizer on the incidence side, the luminescence intensity of a luminescence component having a vibration direction parallel to that on the excitation side can be measured. When a polarizer is arranged on the detection side in a direction orthogonal to the polarizer on the incidence side, the luminescence intensity of a luminescence component having a vibration direction orthogonal to that of the excitation light can be measured. The luminescence intensity may be measured with a spectrophotometer or the like.

The measurement is preferably performed in a dispersion solution at any one temperature within the range of from 0°C to 100°C. The measurement is more preferably performed within the range of from 4°C to 50°C. In addition, the solution is preferably an aqueous solvent, and is, for example, a buffer solution, physiological saline, or water.

Conditions for the measurement may be appropriately set by a person skilled in the art, and Examples and the like to be described later herein may be referred to in setting the conditions, but the conditions are not limited thereto.

In order to generate a sufficient change in polarization anisotropy before and after the aggregation of the particles, it is desired that the first particle be not excessively large. Meanwhile, when the first particle is excessively small, there may arise a problem in that the aggregation via the target substance does not sufficiently occur, or a sufficient luminescent property is not shown, or a problem in production. The average particle diameter of the first particle is preferably 10 nm or more and 1 μm or less. That is, the average particle diameter of the first particle is preferably 1 micron (μm) or less, more preferably 500 nm or less, still more preferably 400 nm or less, still more preferably 250 nm or less. The average particle diameter of the first particle is preferably 10 nm or more, more preferably 20 nm or more, still more preferably 50 nm or more. The term "average particle diameter" as used herein refers to a number-average particle diameter, and the average particle diameter may be measured by a dynamic light scattering method.

The first particle preferably has a small particle size distribution, and preferably has a pdi of 0.1 or less. In addition, the first particle may contain polystyrene and a polymer having a siloxane bond, and contain a hydrophilic polymer in the surface of the first particle. In addition, the first particle may have a layer containing a hydrophilic polymer containing any one of an ether, a betaine, and a pyrrolidone ring on the surface of the particle. When the hydrophilic polymer is contained in the particle surface, nonspecific adsorption is suppressed. When the suppression of nonspecific adsorption is performed not with a protein or the like but with a hydrophilic polymer as described above, the particle size distribution is suppressed, and hence the first particle is more advantageously used for measurement based on polarization anisotropy.

The second particle refers to a particle having a larger average particle diameter than that of the first particle and having affinity for the target substance. The second particle preferably has an average particle diameter of 100 nm or more and 5 μm or less. That is, with regard to the lower limit of the average particle diameter of the second particle, in order to make the polarization anisotropy of the aggregate sufficiently large, the lower limit is preferably 100 nm or more, more preferably 150 nm. The upper limit is preferably 5 μm or less, more preferably 1 μm or less from the viewpoint of, for example, the dispersion stability of the particle.

In addition, the second particle preferably has a small particle size distribution, and preferably has a pdi of 0.1 or less. The second particle may contain polystyrene and a polymer having a siloxane bond, and contain a hydrophilic polymer in the surface of the second particle. In addition, the second particle may have a layer containing a hydrophilic polymer containing any one of an ether, a betaine, and a pyrrolidone ring on the surface of the particle. When the hydrophilic polymer is contained in the particle surface, nonspecific adsorption is suppressed. When the suppression of nonspecific adsorption is performed not with a protein or the like but with a hydrophilic polymer as described above, the particle size distribution is suppressed, and hence the second particle is more advantageously used for measurement based on polarization anisotropy.

There may be adopted such a configuration that the excitation wavelength of the first particle and the excitation wavelength of the second particle differ from each other, or the luminescence wavelength of the first particle and the luminescence wavelength of the second particle differ from each other. Alternatively, there may be adopted such a configuration that the excitation wavelength of the first particle and the excitation wavelength of the second particle differ from each other, and the luminescence wavelength of the first particle and the luminescence wavelength of the second particle differ from each other. That is, the second particle can generate luminescence at an excitation wavelength different from the excitation wavelength of the first particle, and can generate luminescence at a luminescence wavelength different from that of the first particle. When the second particle is provided with luminescence characteristics different from those of the first particle, multidimensional measurement involving, for example, excitation at a plurality of wavelengths or observation of luminescence at a plurality of wavelengths can be performed.

Meanwhile, it is desired that the second particle be not excited at a wavelength suited for exciting the first particle. Specifically, the second particle is preferably free from being excited by excitation light having a wavelength of 300 nm or more and 450 nm or less.

In addition, the second particle is preferably free from generating luminescence that overlaps that of the first particle. Specifically, the second particle is preferably free of a luminescence maximum in a wavelength region of from 550 nm or more to 650 nm or less.

The first particle and the second particle may each have a ligand in order to have affinity for the target substance. Any ligand that shows affinity for a particular substance may be used as the ligand. Examples of a combination of the ligand and the target compound, or the target compound and the ligand may include the following. That is, the examples may include: an antigen and an antibody; a low-molecular-weight compound and a receptor thereof; an enzyme and a substrate; and nucleic acids complementary to each other. Further, the examples may include an antibody and any of the following substances specific thereto: an allergen, a bacterium, a virus, a cell, a cell membrane constituent, a cancer marker, various disease markers, an antibody, a blood-derived substance, a food-derived substance, a natural product-derived substance, and any low-molecular-weight compound. Further, the examples may include a receptor and any of the following substances specific thereto: a low-molecular-weight compound, a cytokine, a hormone, a neurotransmitter, a transmitter, and a membrane protein. Further, the examples may include DNA, RNA, or cDNA derived from a bacterium, a virus, or a cell, a part or fragment thereof, a synthetic nucleic acid, a primer, or a probe, and a nucleic acid having complementarity thereto. Other than the foregoing, a combination known to have affinity is used as the combination of the target substance and the ligand. Particularly typical examples of the ligand may include an antigen, an antibody, and a nucleic acid.

The ligand may be introduced into the first particle or the second particle in the following manner. That is, after the substrate of the particle or a layer for forming the surface thereof has been formed, the ligand may be bonded via a functional group that the substrate or the layer has. Alternatively, the ligand may be incorporated at the time of the production of the substrate of the particle or the layer for forming the surface thereof. A ligand to be used for a latex agglutination method or the like may be diverted and used as the ligand.

The present invention also provides, as a further embodiment thereof, test kit characterized in that the first particle and the second particle are dispersed in a dispersion medium. The present invention also provides a method of producing the test kit, the method including a step of dispersing the first particle and the second particle in a dispersion medium. The test kit may be enclosed in a case.

The present invention also provides, as one embodiment thereof, a method of producing the first particle, the method including at least a first step and a second step. In the first step, a rare earth complex, a radically polymerizable monomer including styrene, a radical polymerization initiator, and a polymer containing a unit having a pyrrolidone ring are mixed with an aqueous medium to prepare an emulsion. In the second step, the radically polymerizable monomer is polymerized by stirring the emulsion obtained in the first step.

A particle that binds to the target substance is sometimes referred to as "affinity particle", a particle showing luminescence is sometimes referred to as "luminescent particle", and a particle having a relatively large average particle diameter is sometimes referred to as "large-particle-diameter particle". A particle that has a luminescent property and binds to the target substance is sometimes referred to as "luminescent affinity particle". In addition, a particle that has a relatively large average particle diameter and binds to the target substance is sometimes referred to as "large-particle-diameter affinity particle". The first particle and the second particle in the present invention correspond to the luminescent affinity particle and the large-particle-diameter affinity particle, respectively.

By reducing the particle size distribution of particles and introducing a luminescent rare earth complex into the particles, a minute change in dispersion state of the particles in a liquid can be grasped as a change in polarized luminescence characteristic. That is, when particles having affinity aggregate in such a manner as to sandwich the target substance therebetween (a sandwich-type reaction occurs), a change in rotational Brownian motion of the particles is generated. This can be grasped as a change in polarization anisotropy. According to this method, the target substance can be detected even in an amount of from about a nanogram to about a picogram per mL of solution.

The rare earth complex can emit polarized light that is phosphorescence having a long lifetime. The polarization anisotropy is related to the anisotropy of a luminescent dye in transition moment (transition dipole moment). In the case of a luminescent dye having anisotropy in transition moment, when polarized light along its transition moment is used as excitation light, its luminescence is also polarized light along the transition moment. In the case of the rare earth complex, when a ligand having anisotropy is excited, fluorescent luminescence based on energy transfer from the ligand to the central metal ion is shown, and hence polarized light is emitted when the ligand is excited with polarized light.

The principle of fluorescence depolarization is the measurement of a shift in transition moment based on the rotational motion of a luminescent material during the occurrence of polarized luminescence. The rotational motion of the luminescent material may be expressed by the equation 3:

$$Q = 3V\eta/kT \cdots \text{(Equation 3)}$$

where Q represents the rotational relaxation time of the material, V represents the volume of the material, $\eta$ represents

the viscosity of a solvent, "k" represents the Boltzmann constant, and T represents an absolute temperature.

The rotational relaxation time of the material is a period of time required for a molecule to rotate by an angle $\theta$ (68.5°) at which $\cos\theta=1/e$.

It is found from the equation 3 that the rotational relaxation time of the luminescent material is proportional to the volume of the material, that is, the cube of the particle diameter of the luminescent material. Meanwhile, a relationship between the luminescence lifetime of the material and the degree of polarization in the fluorescence depolarization may be expressed by the equation 4:

$$p0/p=1+A(\tau/Q) \cdots \text{(Equation 4)}$$

where p0 represents a degree of polarization at a time when the material is stationary (Q=∞), "p" represents the degree of of polarization, A represents a constant, $\tau$ represents the luminescence lifetime of the material, and Q represents the rotational relaxation time.

According to the equations 3 and 4, in order for a change in degree of polarization to be measured large, a relationship between the luminescence lifetime of the luminescent material and its rotational relaxation time, that is, the volume (particle diameter) of the luminescent material, is important, and as the particle diameter of the luminescent material increases, the luminescence lifetime needs to be lengthened as well.

When the degree of polarization of the luminescence represented by the equation 4 is determined experimentally, it is appropriate that polarized light be allowed to enter a sample, and luminescence be detected in a 90° direction with respect to the traveling direction and vibration direction of excitation light. In this case, it is appropriate that the detected light be detected by being divided into polarized light components in parallel and perpendicular directions with respect to the polarized light that is the incident light, and be evaluated according to the equation 5:

$$r(t)=(I\|(t)-GI⊥(t))/(I\|(t)+2GI⊥(t)) \cdots \text{(Equation 5)}$$

where r(t) represents a polarization anisotropy at a time "t", I‖(t) represents the luminescence intensity of a luminescence component parallel to the excitation light at the time "t", I⊥(t) represents the luminescence intensity of a luminescence component perpendicular to the excitation light at the time "t", and G represents a correction value, the ratio of I⊥/I‖ measured with excitation light having a vibration direction different by 90° from that of the excitation light used for sample measurement.

That is, when the particle (or aggregate) size and the luminescence lifetime fall within appropriate ranges, a change in size of the luminescent material through an antigen-antibody reaction or the like can be sensitively read as a value of the polarization anisotropy. The polarization anisotropy refers to a value corrected with G and 2G, and the degree of polarization is a value obtained by removing G and 2G from the equation 5. In actual measurement, the correction value G is required, and hence the polarization anisotropy is determined.

As a material design, it is appropriate that the first particle be small, and that the particle size increase as the polarization anisotropy becomes more saturated through a reaction with the target substance. However, when a sandwich-type antigen-antibody reaction is performed with only one kind of particles, the size of a particle to be formed after the reaction is limited. When the size of the first particle is increased to increase the polarization anisotropy, the anisotropy before the reaction is increased, and the change in polarization anisotropy cannot be increased after all.

In view of the foregoing, attention has been focused on the following fact: when a sandwich-type reaction can be performed between the first particle and the second particle having a larger average particle diameter than that of the first particle, the difference in polarization anisotropy before and after the reaction is increased.

(Detailed Description of Particles)

An example of the particles according to this embodiment is described in detail with reference to the drawings.

(First Particle 1)

A first particle (luminescent affinity particle) 1 according to this embodiment has a small particle size distribution, and the surface of the particle is hydrophilically coated. A luminescent rare earth complex is present inside the particle.

FIG. 1 is a schematic view for illustrating an example of the first particle and the second particle according to this embodiment. In FIG. 1, an example in which the particles are spherical is illustrated. The diameter (average particle diameter) of the first particle is preferably 10 nm or more, or 20 nm or more, more preferably 25 nm or more, and is

preferably 1 μm or less, more preferably 400 nm or less. The average particle diameter of the first particle in this embodiment is particularly preferably 20 nm or more and 400 nm or less. The particle size distribution is preferably more uniform. For example, a particle having a polydispersity index (hereinafter abbreviated as "pdi") of 0.1 or less as measured by a dynamic light scattering method may be suitably used.

The pdi may be determined by irradiating particles dispersed in a solution with laser light, and observing the resultant scattered light with a photon detector. The particles are constantly moving by Brownian motion, and hence an intensity distribution based on interference of the scattered light also fluctuates constantly. The dynamic light scattering method is a particle measurement method for observing the state of the Brownian motion as a fluctuation in scattered light intensity. The fluctuation of scattered light with respect to time is expressed as an autocorrelation function, and a translational diffusion coefficient is determined. A Stokes diameter is determined from the determined diffusion coefficient, and the size of each of the particles dispersed in the solution can be derived. In addition, when the autocorrelation function is subjected to cumulant analysis, a cumulant diameter and the pdi can be determined. The pdi indicates the polydispersity of a particle diameter distribution, and a smaller numerical value thereof indicates that the particle size distribution is more uniform. It is considered that, in general, particles having a pdi of 0.1 or less are monodispersed in a solvent.

The first particle 1 according to this embodiment suitably has a pdi of 0.1 or less. In order to stably adjust the pdi to 0.1 or less, it is desired that a nonspecific adsorption-suppressing agent be not provided on the surface of the particle. Meanwhile, for the purpose of the present invention, nonspecific adsorption of a substance except the target substance onto the particle needs to be prevented, and to that end, a coating for keeping the surface of the particle hydrophilic is needed.

Meanwhile, it is difficult to stably keep the pdi at 0.1 or less by the supporting of BSA, which is generally performed for the above-mentioned purpose. In addition, the supporting of BSA may cause a lot-to-lot variation. In view of this, such a coating that the pdi for the particle size becomes 0.1 or less through use of a hydrophilic polymer is needed.

The first particle according to this embodiment preferably has a diameter of 10 nm or more and 1 μm or less. The diameter is more suitably 20 nm or more and 500 nm or less. Further, the diameter is more suitably 50 nm or more and 400 nm or less, and the diameter is still more suitably 50 nm or more and 250 nm or less. When the average particle diameter is more than 1 μm, the polarization anisotropy before aggregation is increased to reduce the difference from the polarization anisotropy after an aggregation reaction. In addition, when the average particle diameter is less than 10 nm, the volume per particle is reduced to reduce the amount of the rare earth complex that can be incorporated, with the result that a luminescence intensity required for measurement is not obtained in some cases.

(Particle Substrate 2)

A particle substrate 2 refers to the core portion of the first particle 1. The particle substrate 2 preferably has a spherical shape or a shape close thereto. A material for the particle substrate 2 is not particularly specified as long as the material can stably contain the rare earth complex. As the particle substrate, in particular, there may be suitably used, for example, a polystyrene particle obtained by polymerizing a styrene monomer as a main component, or a particle obtained by incorporating a polymer having a siloxane bond into the polystyrene particle. The polystyrene particles may be produced as particles having an extremely uniform particle size distribution by an emulsion polymerization method to be described later. In addition, a hydrophilic coating to be described later or a ligand may be provided via a silanol present in the polymer having a siloxane bond.

(Hydrophilic Layer 3)

A hydrophilic layer 3 is formed on the outside of the particle substrate 2, and includes a molecule or polymer containing a hydrophilic group. The "molecule containing a hydrophilic group" means a molecule or polymer having a hydroxy group, an ether, pyrrolidone, a betaine structure, or the like. Specifically, the hydrophilic layer 3 may contain as a main component, for example, polyethylene glycol, polyvinylpyrrolidone, a polymer of sulfobetaine or phosphobetaine, or polyglycidyl methacrylate whose molecule has an end modified with a hydroxy group by ring-opening a glycidyl group. The hydrophilic layer 3 may be provided as single molecules each having a hydrophilic group on the surface of the silica particle 1 through use of a silane coupling agent or the like, or, as described later, may be formed simultaneously with the synthesis of the particle substrate 2. The hydrophilic layer 3 has no limitation on its thickness, and only needs to be able to exhibit hydrophilicity. However, when the hydrophilic layer is excessively thick, the hydrophilic layer may become hydrogel-like and be hydrated by the influence of ions in a solvent, leading to its destabilization through a change in thickness. The thickness of the hydrophilic layer is suitably 1 nm or more and 50 nm or less.

(Rare Earth Complex 4)

A luminescent rare earth complex is preferably used as a luminescent dye in the present invention because of the following feature: the wavelength and intensity of its luminescence are hardly influenced by the surroundings, and hence the luminescence has a long lifetime. A rare earth complex 4 includes a rare earth element and a ligand. The rare earth element is selected from europium, terbium, neodymium, erbium, yttrium, lanthanum, cerium, samarium, gadolinium, dysprosium, thulium, ytterbium, scandium, and the like. In consideration of the luminescence lifetime and the presence of a visible luminescence wavelength region or the like, europium or terbium is particularly suitably used. For example, europium generally has a luminescence lifetime of from 0.1 ms to 1.0 ms. It is preferred that the luminescence lifetime and the rotational relaxation time obtained from the equation 3 be appropriately adjusted. In the case of europium in a water dispersion, when the particle size of the first particle is set to a diameter of from about 50 nm to about 300 nm, the polarization anisotropy represented by the equation 5 significantly changes before and after aggregate formation.

At least one of the constituent ligands of the rare earth complex 4 needs to be a ligand having a light-collecting function. The "light-collecting function" refers to an action of being excited at a particular wavelength to excite the central metal of the complex through energy transfer. Further, the ligand having a light-collecting function is preferably a molecule having anisotropy in transition moment, and for example, phenanthroline is suitably used. However, the ligand having a light-collecting function is not limited thereto. In addition, it is preferred that the constituent ligands of the rare earth complex 4 include a ligand such as a β-diketone to prevent coordination of a water molecule. The ligand such as the β-diketone coordinated to a rare earth ion suppresses a deactivation process due to the transfer of energy to a solvent molecule or the like to provide strong fluorescence luminescence.

The rare earth complex 4 may be a polynuclear complex as long as the complex has anisotropy in transition moment.

At the time of a state in which the Brownian rotational motion of the rare earth complex 4 can be regarded as stationary in a medium, the polarization anisotropy is desirably 0.1 or more. The state in which the Brownian rotational motion can be regarded as stationary refers to a state in which the rotational relaxation time of the particles is sufficiently longer than the luminescence lifetime of the rare earth complex 4.

(Ligand 5)

In this embodiment, the "ligand" refers to a compound that specifically binds to the target substance or part thereof. The ligand has selective or specific affinity for the target substance. Examples of a combination of the target substance and the ligand, or the ligand and the target substance include, but not limited to: an antigen and an antibody; an enzyme protein and a substrate thereof; a signal substance, such as a hormone or a neurotransmitter, and a receptor thereof; and a nucleic acid and another nucleic acid. Typical examples of the nucleic acids include deoxyribonucleic acids. In FIG. 1, a ligand 5 is included in each of a second particle 7 and the first particle 1.

The ligand 5 may be bonded via a functional group present on the particle substrate 2 or the hydrophilic layer 3 and capable of bonding the ligand, or the ligand may be incorporated at the time of the production of the particle substrate or the formation of the hydrophilic layer.

In addition, with reference to a known ligand to be used for a latex agglutination method, a similar product may be used as the ligand 5. A ligand that causes aggregation in the latex agglutination method is highly likely to be effectively used in this embodiment as well.

Typical examples of the ligand in this embodiment may include an antibody, an antigen, and a nucleic acid.

(Target Substance 6)

A target substance 6 in FIG. 1 is a measurement object, and is a substance that shows affinity for the ligand 5 and is specifically adsorbed thereonto. The target substance 6 may be any substance as long as a ligand corresponding thereto is available. Examples of the target substance may include the following. That is, the examples may include an antibody, an antigen, a nucleic acid, a low-molecular-weight compound, various receptors, an enzyme, and a substrate. Further, the examples may include any substances, such as an allergen, a cytokine, a hormone, a bacterium, a virus, DNA, RNA, cDNA, a cell, a cell membrane constituent, a cancer marker, various disease markers, an antibody, a blood-derived substance, a food-derived substance, and a natural product-derived substance. The target substance 6 reacts with the ligand 5 of each of the first particle 1 and the second particle 7. Thus, an aggregate 8 is formed via the target substance 6. In FIG. 1, an example with one second particle 7, one target substance 6, and one first particle 1 is schematically illustrated. In actuality, the first particle 1 and the second particle 7 both have a large number of ligands and bind to a large number of the target substances 6, via which the particles are further bound with a large number of first particles 1 and/or second particles to form the aggregate 8.

(Second Particle 7)

The second particle (large-particle-diameter affinity particle) 7 according to this embodiment includes the particle substrate 2, the hydrophilic layer 3, and the ligand 5 similar to those of the first particle 1, but does not need to contain the rare earth complex 4.

The second particle 7 may have properties similar to those of the first particle except for not containing the rare earth complex 4 and being different in particle size.

As the second particle 7 becomes larger, the effect of generating a change in polarization anisotropy by aggregate formation becomes higher. Meanwhile, when the particle size is excessively large, dispersion stability in a liquid is reduced, and hence the particle may be sedimented. In addition, when the particle is excessively large, light scattering per particle is enhanced, and hence polarized light may be depolarized. Accordingly, the average particle diameter of the second particle 7 is preferably 100 nm or more and 5 $\mu$m or less.

The second particle 7 is preferably free of luminescence or absorption of such a degree as to interfere with measurement at the wavelength for measuring the polarization anisotropy or in a wavelength region in which the rare earth complex 4 showing polarization anisotropy is excited.

When a liquid having dispersed therein the first particle 1 and the second particle 7 according to this embodiment is used, the anisotropy of polarized luminescence can be detected with high sensitivity with respect to the aggregation/dispersion behavior of the particles. Accordingly, a colloidal liquid obtained by dispersing the first particle 1 and the second particle 7 in an aqueous solvent can be utilized as a high-sensitivity test reagent through use of a fluorescence depolarization method. A buffer solution may be used as the aqueous solvent. In addition, a surfactant, a preservative, a sensitizer, or the like may be added into the aqueous solvent in order to enhance the stability of the dispersion.

(Example of Method of producing Luminescent Particle)

Next, an example of a method of producing the luminescent particle according to this embodiment is described.

The luminescent particle according to this embodiment is produced as described below. A radically polymerizable monomer, a radical polymerization initiator, a rare earth complex, and a hydrophilic polymer are mixed with an aqueous medium to prepare an emulsion (first step).

Further, the emulsion is heated to polymerize the monomer (second step).

The luminescent particle according to this embodiment is a luminescent particle obtained by copolymerizing a compound having a double bond having radical polymerizability.

As appropriate, the method may further include a step (third step) of providing a functional group for bonding a ligand on the surface of the luminescent particle. Herein, any one of a carboxy group, an amino group, a thiol group, an epoxy group, a maleimide group, a succinimidyl group, or a silicon alkoxide group may be used as the functional group capable of bonding a ligand.

(1. Radically Polymerizable Monomer)

The radically polymerizable monomer may include a styrene-based monomer. The radically polymerizable monomer may further include a monomer selected from the group consisting of: an acrylate-based monomer; and a methacrylate-based monomer. Examples of such monomer may include butadiene, vinyl acetate, vinyl chloride, acrylonitrile, methyl methacrylate, methacrylonitrile, and methyl acrylate. In addition to the styrene-based monomer, at least one selected from the group of the above-mentioned monomers may be used. That is, those monomers may be used alone or in combination thereof. In addition, a monomer having two or more double bonds per molecule such as divinylbenzene may be used as a crosslinking agent.

In addition, a radically polymerizable monomer containing an organic silane may be further used in order to incorporate a siloxane bond into the luminescent particle. An organic silane compound may be, for example, vinyltrimethoxysilane, vinyltriethoxysilane, p-styryltrimethoxysilane, 3-methacryloxypropylmethyldimethoxysilane, 3-methacryloxypropyltrimethoxysilane, 3-methacryloxypropylmethyldiethoxysilane, 3-methacryloxypropyltriethoxysilane, or 3-acryloxypropyltrimethoxysilane.

At least one selected from the group of those monomers may be used. That is, those organic silane compounds may be used alone or in combination thereof. The radically polymerizable monomer containing an organic silane forms a backbone of an inorganic oxide in the synthesized luminescent particle to improve the physical and chemical stability of the luminescent particle. Further, the radically polymerizable monomer containing an organic silane enhances affinity between a unit having a functional group capable of bonding the hydrophilic polymer on the surface of the luminescent particle to a ligand and a backbone material for a polymer fine particle including styrene.

When a siloxane bond is incorporated into the luminescent particle, the ratio of the unit having a siloxane bond to the styrene monomer is preferably 40 mass% or less.

Further, the use of the radically polymerizable monomer containing an organic silane can provide a silanol group on the surface of the luminescent particle. With a hydrogen bond between the silanol group and the polymer showing hydrophilicity such as PV polyvinylpyrrolidone, the polyvinylpyrrolidone is more strongly adsorbed onto the particle surface.

(2. Radical Polymerization Initiator)

A wide range of compounds selected from, for example, azo compounds and organic peroxides may each be used as the radical polymerization initiator. Specific examples of the radical polymerization initiator may include 2,2'-azobis(isobutyronitrile), 2,2'-azobis(2,4-dimethylvaleronitrile), 2,2'-azobis(2-methylbutyronitrile), 4,4'-azobis(4-cyanovaleric acid), 2,2'-azobis(2-methylpropionamidine) dihydrochloride, dimethyl 2,2'-azobis(2-methylpropionate), tert-butyl hydroperoxide, benzoyl peroxide, ammonium persulfate (APS), sodium persulfate (NPS), and potassium persulfate (KPS).

(3. Hydrophilic Polymer)

Examples of the hydrophilic polymer for suppressing nonspecific adsorption include hydrophilic polymers each containing a unit having an ether, a betaine, a pyrrolidone ring, or the like. The hydrophilic polymer is contained in the synthesized luminescent particle, and may be mainly present on the surface of the particle. As a preferred example, a case in which a polymer having a pyrrolidone ring (hereinafter sometimes abbreviated as "PVP") is used is described.

The PVP may be fed at the time of the synthesis of the particle, and the addition of the PVP can simultaneously impart a nonspecific adsorption-suppressing ability and a ligand-bonding ability to the particle. The PVP is more hydrophilic than the radically polymerizable monomer, and hence the PVP is present at an interface between the solvent and the particle that is being produced at the time of the synthesis. The PVP is adsorbed onto the surface of the particle by involving part of the polymer chain of the PVP at the time of the polymerization, or by physical/chemical adsorption such as an interaction between a pyrrolidone ring and styrene (radically polymerizable monomer).

The molecular weight of the PVP is preferably 10,000 or more and 100,000 or less, more suitably 40,000 or more and 70,000 or less. When the molecular weight is less than 10,000, the hydrophilicity of the surface of the luminescent particle is weak, and hence nonspecific adsorption is liable to occur. When the molecular weight is more than 100,000, the hydrophilic layer of the surface of the luminescent particle becomes excessively thick, with the result that the luminescent particle gelates and becomes difficult to handle.

At the time of the synthesis of the luminescent particle, another hydrophilic polymer may be added as a protective colloid in addition to the PVP or separately from the PVP.

In an example of the luminescent particle prepared as described above, 30 μL of a dispersion of 0.1 mass% of the particle was added to 60 μL of a buffer solution mixed with 16 μL of serum, and the whole was left to stand at 37°C for 5 minutes. A difference in absorption spectrum at an optical path of 10 mm and a wavelength of 572 nm before and after the addition was 0.1 or less. That is, the nonspecific adsorption of impurities in serum was sufficiently low.

(4. Aqueous Medium)

The aqueous solution (aqueous medium) to be used at the time of the above-mentioned synthesis preferably contains water at 80% or more and 100% or less in the medium. Examples of the aqueous solution include water and a solution obtained by mixing water with a water-soluble organic solvent, such as methanol, ethanol, isopropyl alcohol, or acetone. When an organic solvent other than water is incorporated at more than 20%, the dissolution of the polymerizable monomer may occur at the time of the production of the luminescent particle.

In addition, when the radically polymerizable monomer containing an organic silane is used, the aqueous solution (aqueous medium) preferably has its pH adjusted to 6 or more and 9 or less in advance. When the pH has a value of less than 6 or more than 9, an alkoxide group or a silanol group in the organic silane compound may undergo condensation polymerization or a reaction with another functional group before the formation of the luminescent particle, leading to aggregation of the particles to be obtained. In this embodiment, the alkoxide is not intentionally subjected, before the formation of the particle, to condensation polymerization.

The pH is preferably adjusted with a pH buffer, but may be adjusted with an acid or a base. Other than the foregoing, a surfactant, an antifoaming agent, a salt, a thickener, and the like may be used by being added at a ratio of 10% or less with respect to the aqueous medium.

In the production of the polymer particle according to this embodiment, it is preferred that, first, the PVP be dissolved in the aqueous medium adjusted to a pH of from 6 to 9. The PVP is added in an amount of from 0.01 mass% to 10 mass%, preferably from 0.03 mass% to 5 mass% with respect to the aqueous medium. When the amount is 0.01 mass% or less, the amount of adsorption onto the polymer fine particle is small, and the effect thereof is not expressed. In addition, when the amount is 10 mass% or more, the viscosity of the aqueous medium may be increased to preclude sufficient stirring.

Subsequently, a radically polymerizable monomer (A) is added into the above-mentioned aqueous medium to prepare an emulsion. At this time, the emulsion may be prepared by also adding a radically polymerizable monomer (B) containing an organic silane into the aqueous medium in addition to the (A). A mass ratio between the (A) and the (B) is from 6:4 to 10:0. Further, the prepared monomer liquid is mixed with a luminescent rare earth complex. At this time, when the solubility of the luminescent rare earth complex is low, a water-insoluble organic solvent may be added. A mass ratio between the luminescent rare earth complex and the monomer(s) is from 1:1,000 to 1:10.

When the (A) is 60% or less, the specific gravity of the particle as a whole may be increased, resulting in remarkable sedimentation of the particle. In addition, the (B) may be absent, but it is more suitable to add the (B) in order to increase adhesiveness between the PVP and the luminescent particle.

A mass ratio between the aqueous medium and the total amount of the (A) and the (B) is preferably from 5:5 to 9.5:0.5. When the ratio of the aqueous medium is 50% or less, nonspecific aggregation of the particles to be produced may become a problem. In addition, even when the ratio is 95% or more, there is no problem with the production of the luminescent particle, but the production amount thereof may be reduced.

The radical polymerization initiator is used by being dissolved in water, a buffer, or the like. The radical polymerization initiator may be used between 0.5 mass% and 10 mass% with respect to the total mass of the (A) and the (B).

In the above-mentioned step of heating the emulsion, it is only required that the entire emulsion be uniformly heated. A heating temperature may be arbitrarily set between 50°C and 80°C, and a heating time may be arbitrarily set between 2 hours and 24 hours. Through the heating of the emulsion, the monomers are polymerized.

The functional group capable of bonding a ligand is preferably a functional group capable of bonding, for example, a protein, a nucleic acid, a peptide, an amino acid, or an amino group. Examples of such functional group include a carboxy group, an amino group, a thiol group, an epoxy group, a maleimide group, a succinimidyl group, and a silicon alkoxide group. A silane coupling agent having the functional group capable of bonding a ligand and the synthesized luminescent particle may be mixed to provide the functional group on the luminescent particle surface. Specifically, for example, an aqueous solution of a silane coupling agent having a carboxy group may be prepared and mixed with a dispersion of the synthesized luminescent particle to provide the carboxy group on the particle surface. At this time, a dispersant such as Tween 20 may be added to the reaction solution. A reaction temperature may be arbitrarily set between 0°C and 80°C, and a reaction time may be arbitrarily set between 1 hour and 24 hours. In order to suppress an abrupt condensation reaction of the silane coupling agent, it is suitable that the temperature be set to be equal to or lower than a room temperature of about 25°C, and the reaction time be set to from about 3 hours to about 14 hours. Depending on the functional group capable of bonding a ligand, the reaction with the particle surface may be promoted by adding an acid or alkali catalyst.

Affinity for the target substance may be imparted to the particle synthesized through emulsion polymerization or the like by bonding a ligand such as any of various antibodies thereto. A technique for bonding the ligand is not particularly limited, and an optimal technique for bonding an antibody of interest through utilization of a functional group present on the hydrophilic layer 3 or the particle substrate 2 only needs to be selected.

(Method of producing Large-particle-diameter Particle)

The large-particle-diameter particle may be obtained in the same manner as in the above-mentioned synthesis method for the luminescent particle excluding the step of introducing the rare earth complex 4.

However, the large-particle-diameter particle may contain a fluorescent dye different from the rare earth complex 4.

(Affinity Particles)

In this embodiment, the "ligand" refers to a compound that specifically binds to a particular target substance. The ligand binds to the target substance at a predetermined site and has selectively or specifically high affinity. For example, typical examples of a combination of the target substance and the ligand, or the ligand and the target substance may include the following. That is, the examples include: an antigen and an antibody; an enzyme protein and a substrate thereof; a signal substance, such as a hormone or a neurotransmitter, and a receptor thereof; and nucleic acids. However, the ligand in this embodiment is not limited thereto. Examples of the nucleic acids include deoxyribonucleic acids. The affinity particles in this embodiment have selective or specific affinity for the target substance. Typical examples of the ligand in this embodiment include an antibody, an antigen, and a nucleic acid.

A hitherto known method may be applied to a chemical reaction method for chemically bonding the reactive functional group that the particle according to this embodiment has and the ligand to the extent that the object of the present invention can be achieved. In addition, when the ligand is amide-bonded, a catalyst such as 1-[3-(dimethylaminopropyl)-3-ethylcarbodiimide] may be appropriately used.

When using an antibody (antigen) as the ligand and an antigen (antibody) as the target substance, the affinity particles in this embodiment may be preferably applied to a latex immunoagglutination measurement method utilized widely in the fields of clinical tests, biochemical research, and the like.

(Test Kit)

The test kit according to this embodiment includes the first particle and the second particle. Further, the test kit may include a dispersion medium. In addition, in the test kit, the first particle and the second particle may be dispersed in the dispersion medium. In addition, the test kit may include a reagent containing the first particle, the second particle, and the dispersion medium. The amount of the affinity particles according to this embodiment to be incorporated into the reagent in this embodiment is preferably from 0.000001 mass% to 20 mass%, more preferably from 0.0001 mass% to 1 mass%. The reagent according to this embodiment may include, in addition to the affinity particles according to this embodiment, a third substance, such as a solvent or a blocking agent, to the extent that the object of the present invention can be achieved. The reagent may include a combination of two or more kinds of third substances, such as a solvent and a blocking agent. Examples of the solvent to be used in this embodiment include various buffer solutions, such as a phosphate buffer solution, a glycine buffer solution, a Good's buffer solution, a Tris buffer solution, and an ammonia buffer solution, but the solvent in the reagent in this embodiment is not limited thereto.

The test kit may include the above-mentioned reagent and a case enclosing the reagent.

In addition, the kit according to this embodiment may contain a sensitizer for promoting the aggregation of the particles via the target substance. Examples of the sensitizer include polyvinyl alcohol, polyvinylpyrrolidone, and polyalginic acid, but the present invention is not limited thereto. In addition, the kit according to this embodiment may include a positive control, a negative control, a serum diluent, or the like. As a medium for the positive control or the negative control, there may be used serum free of the target substance, physiological saline, or a solvent. The kit according to this embodiment may be used for the method of detecting a target substance according to this embodiment in the same manner as a kit for use in general detection of a target substance in a specimen by in vitro diagnosis. In addition, the concentration of the target substance may also be measured by a hitherto known method, and in particular, the kit is suitably used for the detection of a target substance in a specimen by a latex agglutination method.

(Detection Method)

The method of determining the presence or absence or concentration of a target substance in this embodiment includes a step of mixing the first particle and the second particle according to this embodiment with a sample liquid that may contain the target substance. The mixing is preferably performed within the range of from pH 3.0 to pH 11.0. In addition, a mixing temperature falls within the range of from 0°C to 100°C, or from 4°C to 50°C, or even from 20°C to 50°C, and a mixing time falls within the range of from 1 second to 2 hours, or from 1 minute to 60 minutes. In addition, this method preferably uses a solvent. In addition, in the method according to this embodiment, the concentration of the first particle and the second particle is preferably as described below. That is, their total amount is preferably from 0.000001 mass% to 1 mass%, more preferably from 0.00001 mass% to 0.001 mass% in a reaction system. In the detection method according to this embodiment, an aggregation reaction occurring as a result of the mixing of the first particle, the second particle, and the specimen is detected by a fluorescence depolarization method.

That is, the detection method specifically includes the steps of: mixing a test reagent with a specimen to provide a mixed liquid; irradiating the mixed liquid with polarized light; and separately detecting polarized light components of luminescence of affinity particles in the mixed liquid.

Through optical detection of the above-mentioned aggregation reaction occurring in the mixed liquid, the target substance in the specimen is detected, and further, the concentration of the target substance may also be measured.

[Examples]

The present invention is specifically described below by way of Examples. However, the present invention is not limited to these Examples.

(1) Production of Luminescent Particles

Polyvinylpyrrolidone (PVP-K30: manufactured by Tokyo Chemical Industry Co., Ltd.) and sodium dodecyl sulfate (manufactured by Wako Pure Chemical Industries, Ltd., hereinafter abbreviated as "SDS") were dissolved in a 2-morpholinoethanesulfonic acid (MES) buffer solution (manufactured by Kishida Chemical Co., Ltd.) having a pH of 7 to prepare a solvent A. Eu(TTA)$_3$Phen manufactured by Central Techno Corporation was used as a rare earth complex. Eu(TTA)$_3$Phen is also expressed as [tris(2-thenoyltrifluoroacetonato)(1,10-phenanthroline)europium(III)].

Eu(TTA)$_3$Phen was mixed with a styrene monomer (manufactured by Kishida Chemical Co., Ltd.) and 3-methacryloxypropyltrimethoxysilane (manufactured by Tokyo Chemical Industry Co., Ltd., hereinafter abbreviated as "MPS") to prepare a reaction liquid B. The reaction liquid B was added to the solvent A in a four-necked flask, and the mixture was stirred with a mechanical stirrer set to 300 rpm. After 30 minutes of stirring under a nitrogen flow condition, the temperature of an oil bath that had been prepared was set to 70°C, and the nitrogen flow was performed for an additional 30 minutes. After the heating and the stirring, an aqueous solution having dissolved therein potassium persulfate (manufactured by Sigma-Aldrich, KPS) was added into the reaction solution, and emulsion polymerization was performed for 10 hours. After the reaction, the resultant suspension was centrifuged, the supernatant was removed, and the precipitate was redispersed with pure water. The operations of centrifugation and redispersion were repeated 3 times to wash the product. The resultant precipitate was redispersed with pure water to provide a luminescent particle dispersion. The weight ratios of the reagents used are as shown in Table 1.

[Table 1]

| Sample | Solvent A | | Reaction liquid B | | | Catalyst |
|---|---|---|---|---|---|---|
| | Solvent amount | PVP-K30 | Eu(TTA)$_3$Phen | Styrene monomer | MPS | KPS |
| Luminescent particles-1 | 160 | 1.3 | 0.05 | 3.2 | 1 | 0.4 |
| Luminescent particles-2 | 80 | 0.7 | 0.015 | 3.2 | 1 | 0.01 |

(2) Production of Large-particle-diameter Particles

Polyvinylpyrrolidone (PVP-K30: manufactured by Tokyo Chemical Industry Co., Ltd.) and sodium dodecyl sulfate (manufactured by Wako Pure Chemical Industries, Ltd., SDS) were dissolved in a 2-morpholinoethanesulfonic acid (MES) buffer solution (manufactured by Kishida Chemical Co., Ltd.) having a pH of 7 to prepare a solvent A. A styrene monomer (manufactured by Kishida Chemical Co., Ltd.) was mixed with 3-methacryloxypropyltrimethoxysilane (manufactured by Tokyo Chemical Industry Co., Ltd., MPS) to prepare a reaction liquid B. The reaction liquid B was added to the solvent A in a four-necked flask, and the mixture was stirred with a mechanical stirrer set to 300 rpm. After 30 minutes of stirring under a nitrogen flow condition, the temperature of an oil bath that had been prepared was set to 70°C, and the nitrogen flow was performed for an additional 30 minutes.
After the heating and the stirring, an aqueous solution having dissolved therein potassium persulfate (manufactured by Sigma-Aldrich, KPS) was added into the reaction solution, and emulsion polymerization was performed for 10 hours. After the reaction, the resultant suspension was centrifuged, the supernatant was removed, and the precipitate was redispersed with pure water. The operations of centrifugation and redispersion were repeated 3 times to wash the product. The resultant precipitate was redispersed with pure water to provide a large-particle-diameter particle dispersion. The weight ratios of the reagents used are as shown in Table 2.

[Table 2]

| Sample | Solvent A | | Reaction liquid B | | Catalyst |
|---|---|---|---|---|---|
| | Solvent amount | PVP-K30 | Styrene monomer | MPS | KPS |
| Large-particle-diameter particles-1 | 80 | 0.7 | 4.2 | 1 | 0.005 |
| Large-particle-diameter particles-2 | 80 | 0.7 | 4.2 | 1 | 0.01 |

Aliquots of the dispersions of the luminescent particles and the large-particle-diameter particles obtained by the emulsion polymerization were taken and added to an aqueous solution having dissolved therein 1 wt% of Tween 20 (manufactured by Kishida Chemical Co., Ltd.). After 10 minutes of stirring, a silane coupling agent having a carboxylic acid as an organic functional group, X12-1135 (manufactured by Shin-Etsu Chemical Co., Ltd.), was added, and the mixture was stirred overnight. After the stirring, the dispersion was centrifuged, the supernatant was removed, and the precipitate was redispersed with pure water. The operations of centrifugation and redispersion were performed 3 or more times to wash the product. The precipitate after the washing was redispersed in pure water to provide a dispersion of luminescent particles and large-particle-diameter particles. A weight ratio among the particles, pure water, and X12-1135 was set to 1:300:2.

(3) Production of Anti-CRP Antibody-modified Affinity Particles

An aliquot of 0.25 mL of the particle dispersion at 1.2 wt% of the synthesized luminescent particles and large-particle-diameter particles was taken, and the solvent was replaced by 1.6 mL of a MES buffer solution having a pH of 6.0. To the particle MES buffer solution, 1-[3-(dimethylamino)propyl]-3-ethylcarbodiimide and N-hydroxysulfosuccinimide sodium were added at 0.5 wt%, and the mixture was subjected to a reaction at 25°C for 1 hour. After the reaction, the dispersion was washed with a MES buffer solution having a pH of 5.0, an anti-CRP antibody was added at 100 $\mu$g/mL, and the anti-CRP antibody was bonded to the particles at 25°C for 2 hours. After the bonding, the particles were washed with a Tris buffer solution having a pH of 8. After the reaction, the particles were washed with a phosphate buffer solution to provide CRP antibody-modified affinity particles having a concentration of 0.3 wt%.
The bonding of the antibody was recognized by measuring the amount of a reduction in antibody concentration in the buffer solution having added thereto the antibody by BCA assay.

(4) Reaction between Affinity Particles and Antigen Solution

A change in fluorescence polarization before and after the obtained CRP antibody-modified affinity particles were mixed with a CRP antigen diluted with a MES buffer solution was observed. An investigation was performed with the CRP antibody-modified affinity particles fixed at 0.0001 mg/mL, and with CRP at an antigen concentration of from 0 pg/mL to 1,000 pg/mL. The observation was performed at normal temperature. A measurement method for fluorescence polarization is described later.

(Example 1)

A dispersion in which a sample corresponding to the synthesized luminescent affinity particles-1 and a sample corresponding to the synthesized large-particle-diameter affinity particles-1 were mixed at a ratio of 9:1 in terms of weight ratio was prepared. The prepared dispersion was mixed with the CRP antigen diluted with a MES buffer solution, and a change in fluorescence polarization before and after the mixing was observed. The total amount of the particles in the dispersion was set to 0.001 mg/mL, and the CRP antigen was set to 1 pg/mL. Measurement was performed at normal temperature.

(Example 2)

The same investigation as in Example 1 was performed except that the sample corresponding to the synthesized luminescent affinity particles-1 and the sample corresponding to the synthesized large-particle-diameter affinity particles-1 were set to a ratio of 5:5 in terms of weight ratio.

(Example 3)

The same investigation as in Example 1 was performed except that the sample corresponding to the synthesized luminescent affinity particles-1 and the sample corresponding to the synthesized large-particle-diameter affinity particles-1 were set to a ratio of 1:9 in terms of weight ratio.

(Example 4)

A dispersion in which a sample corresponding to the synthesized luminescent affinity particles-2 and a sample corresponding to the synthesized large-particle-diameter affinity particles-2 were mixed at a ratio of 5:5 in terms of weight ratio was prepared. The prepared dispersion was mixed with the CRP antigen diluted with a MES buffer solution, and a change in fluorescence polarization before and after the mixing was observed. The total amount of the particles in the dispersion was set to 0.001 mg/mL, and the CRP antigen was set to 10 pg/mL. Measurement was performed at normal temperature.

(Comparative Example 1)

A dispersion of a sample corresponding to the synthesized luminescent affinity particles-1 was prepared. The prepared dispersion was mixed with the CRP antigen diluted with a MES buffer solution, and a change in fluorescence polarization before and after the mixing was observed. The total amount of the particles in the dispersion was set to 0.001 mg/mL, and the CRP antigen was set to 1 pg/mL. Measurement was performed at normal temperature.

(Comparative Example 2)

A dispersion of a sample corresponding to the synthesized large-particle-diameter affinity particles-1 was prepared. The prepared dispersion was mixed with the CRP antigen diluted with a MES buffer solution, and a change in fluorescence polarization before and after the mixing was observed. The total amount of the particles in the dispersion was set to 0.001 mg/mL, and the CRP antigen was set to 1 pg/mL. Measurement was performed at normal temperature.

(Comparative Example 3)

A dispersion of a sample corresponding to the synthesized luminescent affinity particles-2 was prepared. The prepared dispersion was mixed with the CRP antigen diluted with a MES buffer solution, and a change in fluorescence polarization before and after the mixing was observed. The total amount of the particles in the dispersion was set to 0.001 mg/mL, and the CRP antigen was set to 1 pg/mL. Measurement was performed at normal temperature.

(Evaluations of Products)

The products in Examples and Comparative Examples were each subjected to the following evaluations.
The shape of the product was evaluated with an electron microscope (S5500 manufactured by Hitachi High-Technologies Corporation).
The average particle diameter of the product was evaluated by using dynamic light scattering (Zetasizer Nano S manufactured by Malvern).
The concentration of a suspension having the product dispersed therein was evaluated with a mass spectrometer (Thermo plus TG8120 manufactured by Rigaku Corporation).
A fluorescence spectrum of a dispersion containing the product was measured at room temperature with excitation light at 340 nm and with a polarizer placed in an optical path on each of an excitation side and a luminescence side. The measurement was performed with the direction of the polarizer on the excitation side being fixed, and the polarizer on the luminescence side being placed in a direction parallel or perpendicular to that on the excitation side. An apparatus used was a fluorescence spectrophotometer F-4500 manufactured by Hitachi High-Tech Science Corporation. The peak wavelength of observation light for analyzing polarized luminescence was set to 611 nm. The resultant data on polarized luminescence was analyzed with the equation 1 to determine the polarization anisotropy "r".
Nonspecific agglutination suppression evaluation of the product was performed as described below.
60 μl of a human serum solution diluted 15-fold with a buffer solution was added to 30 μl of each luminescent affinity particle dispersion (3 mg/mL) produced in Examples 1 to 4 and Comparative Examples 1 to 3, and the mixture was kept at a temperature of 37°C for 5 minutes. An absorbance at 527 nm was measured before and after the temperature keeping, and the amount of change in absorbance before and after the temperature keeping was measured 3 times.

(Performance Evaluation)

According to the results of the nonspecific aggregation suppression evaluation, the change in absorbance was equal to or less than the specific numerical value in all Examples and Comparative Examples. That is, in all of Examples 1 to 4 and Comparative Examples 1 to 3, the change in absorbance was equal to or less than the specific numerical value, i.e., 0.01 or less, and thus it was recognized that the luminescent particles were capable of suppressing nonspecific adsorption.
The structures and physical properties of the particle materials of Examples 1, 2, and 3, and Comparative Examples 1 and 2 are shown in Table 3.

[Table 3]

|  | Example 1 | Example 2 | Example 3 | Comparative Example 1 | Comparative Example 2 |
|---|---|---|---|---|---|
| Particle diameter of luminescent affinity particles | 98 nm | | | 98 nm | - |
|  | (Luminescent affinity particles-1) | | | | |
| Particle diameter of large-particle-diameter affinity particles | 350 nm | | | - | 350 nm |
|  | (Large-particle-diameter affinity particles-1) | | | | |

(continued)

|  | Example 1 | Example 2 | Example 3 | Comparative Example 1 | Comparative Example 2 |
|---|---|---|---|---|---|
| Weight ratio of luminescent affinity particles | 90 | 50 | 10 | 100 | 0 |
| Weight ratio of large-particle-diameter affinity particles | 10 | 50 | 90 | 0 | 100 |
| Polarization anisotropy "r" after reaction | 0.098 | 0.133 | 0.154 | 0.094 | - |
| Change $\Delta r$ in polarization anisotropy | 0.004 | 0.039 | 0.06 | 0.001 | - |

The luminescent affinity particles-1 of Examples 1, 2, and 3, and Comparative Example 1 had a size of 98 nm, and were also recognized in each example to be monodispersed particles having a pdi value of 0.024, i.e., less than 0.1. The large-particle-diameter particles-1 of Examples 1, 2, and 3, and Comparative Example 2 had a size of 347 nm, and were also recognized in each example to be monodispersed particles having a pdi value of 0.082, i.e., less than 0.1. When their respective values of the polarization anisotropy "r" after reaction and changes $\Delta r$ in polarization anisotropy before and after reaction were compared, it was revealed that Examples, in which the large-particle-diameter affinity particles were mixed, had higher values than those of Comparative Example 1 including no large-particle-diameter affinity particles. Further, it was revealed that the value of the polarization anisotropy "r" after reaction and the change $\Delta r$ in degree of polarization before and after reaction were higher in the order of Examples 3, 2, and 1 having increasing ratios at which the large-particle-diameter affinity particles were mixed. It was revealed that Example 3, which had the largest change in degree of polarization, had a 60-fold value for $\Delta r$ as compared to Comparative Example 1.
Meanwhile, in Comparative Example 2 including no luminescent particles, the polarization anisotropy was not able to be detected.
The structures and physical properties of the particle materials of Example 4 and Comparative Example 3 are shown in Table 4.

[Table 4]

|  | Example 4 | | Comparative Example 3 |
|---|---|---|---|
| Particle diameter of luminescent affinity particles | 207 nm | | |
| | (Luminescent affinity particles-2) | | |
| Particle diameter of large-particle-diameter affinity particles | 275 nm | | - |
| | (Large-particle-diameter affinity particles-2) | | |
| Weight ratio of luminescent affinity particles | 50 | | 100 |
| Weight ratio of large-particle-diameter affinity particles | 50 | | - |
| Polarization anisotropy "r" after reaction | 0.141 | | 0.136 |
| Change $\Delta r$ in degree of polarization | 0.022 | | 0.018 |

The luminescent affinity particles-2 of Example 4 and Comparative Example 3 had a size of 207 nm, and were also recognized in each example to be monodispersed particles having a pdi value of 0.039, i.e., less than 0.1. The large-particle-diameter affinity particles-2 of Example 4 had a size of 275 nm, and were also recognized in each example to be monodispersed particles having a pdi value of 0.082, i.e., less than 0.1. When their respective values of the polarization anisotropy "r" after reaction and changes $\Delta r$ in polarization anisotropy before and after reaction were compared, it was revealed that Example 4, in which the large-particle-diameter affinity particles were mixed, had higher values than those of Comparative Example 3 including no large-particle-diameter affinity particles.
Polarization anisotropy determined by a fluorescence depolarization method depends on a particle size (aggregate size), and is proportional to the cube of the radius of a particle (aggregate). The measurement results according to Examples of the present invention were results obtained by investigating an aggregation reaction for increasing the particle (aggregate) size, and were logical results consistent with the measurement principle of the fluorescence depolarization method.

Thus, it was shown that the measurement method according to the present invention had extremely high detection sensitivity, and the test kit, or the luminescent affinity particle and the large-particle-diameter affinity particle of the present invention enabled high-sensitivity measurement.

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2021-096211 filed on June 8, 2021, the description of which is incorporated herein by reference in its entirety.

[Reference Signs List]

1 first particle (luminescent affinity particle)
2 particle substrate
3 hydrophilic layer
4 rare earth complex
5 ligand
6 target substance
7 second particle (large-particle-diameter affinity particle)
8 aggregate

**Claims**

1. A method of determining at least any one of the presence or absence, and a concentration, of a target substance in a sample liquid, the method comprising the steps of:

   (a) preparing a first particle that specifically binds to the target substance and contains a rare earth complex, and a second particle that has a larger average particle diameter than that of the first particle and specifically binds to the target substance;
   (b) mixing the sample liquid, the first particle, and the second particle to provide a mixed liquid; and
   (c) determining at least any one of the presence or absence, and the concentration, of the target substance from polarization anisotropy of the mixed liquid obtained in the step (b).

2. The method according to claim 1, wherein the polarization anisotropy is determined by the following equation 1:
   [Math. 1]

$$<r> = \frac{I_{VV} - G * I_{VH}}{I_{VV} + 2 * G * I_{VH}} \qquad G = \frac{I_{HV}}{I_{HH}} \qquad (1)$$

   where

   $<r>$ represents the polarization anisotropy,
   $I_{VV}$ represents a luminescence intensity of a luminescence component having a vibration direction parallel to that of a first polarized light beam at a time of excitation by the first polarized light beam,
   $I_{VH}$ represents a luminescence intensity of a luminescence component having a vibration direction orthogonal to that of the first polarized light beam at the time of excitation by the first polarized light beam,
   $I_{HV}$ represents a luminescence intensity of a luminescence component having a vibration direction orthogonal to that of a second polarized light beam having a vibration direction orthogonal to that of the first polarized light beam at a time of excitation by the second polarized light beam,
   $I_{HH}$ represents a luminescence intensity of a luminescence component having a vibration direction parallel to that of the second polarized light beam having a vibration direction orthogonal to that of the first polarized light beam at the time of excitation by the second polarized light beam, and
G represents a correction value.

3. A test kit for determining at least any one of the presence or absence, and a concentration, of a target substance, the test kit comprising:

a first particle that specifically binds to the target substance; and
a second particle that has a larger average particle diameter than that of the first particle and specifically binds to the target substance,
wherein the first particle contains a rare earth complex,
wherein the first particle has an average particle diameter of 10 nm or more and 1 $\mu$m or less, and
wherein the second particle has an average particle diameter of 100 nm or more and 5 $\mu$m or less.

4. The test kit according to claim 3,
wherein the first particle

contains polystyrene and a polymer having a siloxane bond,
contains a hydrophilic polymer in a surface of the first particle, and
contains a rare earth complex inside the first particle.

5. The test kit according to claim 3 or 4, wherein the rare earth complex is a complex of a rare earth selected from the group consisting of: europium; terbium; neodymium; erbium; yttrium; lanthanum; cerium; samarium; gadolinium; dysprosium; thulium; ytterbium; and scandium.

6. The test kit according to any one of claims 3 to 5,

wherein the first particle and the second particle form an aggregate via the target substance,
wherein polarization anisotropy that the aggregate shows in a dispersion medium is increased by 0.004 or more as compared to polarization anisotropy that the first particle shows in the dispersion medium, and
wherein the polarization anisotropies are each defined by the following equation 1:
[Math. 2]

$$< r >= \frac{I_{VV} - G * I_{VH}}{I_{VV} + 2 * G * I_{VH}} \qquad G = \frac{I_{HV}}{I_{HH}} \qquad (1)$$

where

<r> represents the polarization anisotropy,
Ivv represents a luminescence intensity of a luminescence component having a vibration direction parallel to that of a first polarized light beam at a time of excitation by the first polarized light beam,
$I_{VH}$ represents a luminescence intensity of a luminescence component having a vibration direction orthogonal to that of the first polarized light beam at the time of excitation by the first polarized light beam,
$I_{HV}$ represents a luminescence intensity of a luminescence component having a vibration direction orthogonal to that of a second polarized light beam having a vibration direction orthogonal to that of the first polarized light beam at a time of excitation by the second polarized light beam,
$I_{HH}$ represents a luminescence intensity of a luminescence component having a vibration direction parallel to that of the second polarized light beam having a vibration direction orthogonal to that of the first polarized light beam at the time of excitation by the second polarized light beam, and
G represents a correction value.

7. The test kit according to claim 6, wherein the aggregate shows a polarization anisotropy of 0.1 or more in the dispersion medium.

8. The test kit according to any one of claims 3 to 7,

wherein an excitation wavelength of the first particle and an excitation wavelength of the second particle differ from each other, or

wherein a luminescence wavelength of the first particle and a luminescence wavelength of the second particle differ from each other, or

wherein the excitation wavelength of the first particle and the excitation wavelength of the second particle differ from each other, and the luminescence wavelength of the first particle and the luminescence wavelength of the second particle differ from each other.

9. The test kit according to any one of claims 3 to 8, wherein the second particle is free from being excited by excitation light having a wavelength of 300 nm or more and 450 nm or less.

10. The test kit according to any one of claims 3 to 9, wherein the second particle is free of a luminescence maximum in a wavelength region of from 550 nm or more to 650 nm or less.

11. The test kit according to any one of claims 3 to 10, wherein the average particle diameter of the first particle is 20 nm or more and 400 nm or less.

12. The test kit according to any one of claims 3 to 11, wherein the first particle and the second particle each have a ligand that specifically binds to the target substance.

13. The test kit according to claim 12, wherein the ligand is selected from the group consisting of: an antibody; an antigen; and a nucleic acid.

14. The test kit according to any one of claims 3 to 13, wherein at least any one of the first particle and the second particle has, on a particle surface thereof, a layer containing a hydrophilic polymer containing any one of an ether, a betaine, and a pyrrolidone ring.

15. The test kit according to any one of claims 3 to 14, wherein the first particle and the second particle are dispersed in a dispersion medium.

16. The test kit according to any one of claims 3 to 15, wherein the test kit is enclosed in a case.

17. A method of producing the first particle of the test kit of any one of claims 3 to 16, the method comprising at least:

a first step of mixing a rare earth complex, a radically polymerizable monomer including styrene, a radical polymerization initiator, and a polymer containing a unit having a pyrrolidone ring with an aqueous medium to prepare an emulsion; and
a second step of polymerizing the radically polymerizable monomer by stirring the emulsion.

18. A method of producing the test kit of any one of claims 3 to 16, the method comprising a step of dispersing the first particle and the second particle in a dispersion medium.

# FIG. 1

## INTERNATIONAL SEARCH REPORT

| International application No. |
|---|
| **PCT/JP2022/022413** |

### A. CLASSIFICATION OF SUBJECT MATTER

*G01N 21/64*(2006.01)i; *G01N 33/543*(2006.01)i
FI: G01N21/64 A; G01N33/543 525C; G01N33/543 581D; G01N33/543 525U; G01N33/543 501M; G01N33/543 575

According to International Patent Classification (IPC) or to both national classification and IPC

### B. FIELDS SEARCHED

Minimum documentation searched (classification system followed by classification symbols)

G01N21/00-21/83; G01N33/48-33/98

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 7-72155 A (MITSUBISHI CHEMICAL CORPORATION) 17 March 1995 (1995-03-17) paragraphs [0001], [0015], [0027], [0030]-[0037], [0040] | 3, 5-18 |
| Y | | 1-2, 4 |
| X | JP 2000-146975 A (MITSUBISHI CHEMICAL CORPORATION) 26 May 2000 (2000-05-26) paragraphs [0001], [0019], [0044]-[0048] | 3, 5-18 |
| Y | | 1-2, 4 |
| Y | WO 2019/132045 A1 (SEKISUI CHEMICAL COMPANY, LIMITED) 04 July 2019 (2019-07-04) paragraphs [0021], [0022], [0024] | 4 |
| Y | WO 2019/208711 A1 (CANON KABUSHIKI KAISHA) 31 October 2019 (2019-10-31) paragraphs [0039]-[0048], [0055] | 4 |
| Y | JP 2893772 B2 (MITSUBISHI CHEMICAL CORPORATION) 24 May 1999 (1999-05-24) column 5, line 5 to column 6, line 19 | 1-2 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **04 August 2022** | **23 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2022/022413** |

### C.   DOCUMENTS CONSIDERED TO BE RELEVANT

| Category* | Citation of document, with indication, where appropriate, of the relevant passages | Relevant to claim No. |
| --- | --- | --- |
| Y | JP 10-512751 A (NEXSTAR PHARMACEUTICALS, INCORPORATED) 08 December 1998 (1998-12-08)<br>    page 10, line 10 to page 12, line 20 | 1-2 |
| Y | US 2011/0207614 A1 (UNIVERSITY OF NORTH TEXAS HEALTH SCIENCE CENTER AT FORT WORTH) 25 August 2011 (2011-08-25)<br>    paragraphs [0035]-[0038], [0051] | 1-2 |

Form PCT/ISA/210 (second sheet) (January 2015)

## INTERNATIONAL SEARCH REPORT

| International application No. |
| --- |
| **PCT/JP2022/022413** |

**Box No. III    Observations where unity of invention is lacking (Continuation of item 3 of first sheet)**

This International Searching Authority found multiple inventions in this international application, as follows:

(Invention 1) Claims 1-2
    Claims 1-2 have the special technical feature of a "method for determining at least one of the concentration and the presence or absence of a target substance in a sample solution, the method comprising (a) a step for preparing first particles that specifically bind to the target substance and contain a rare-earth complex and second particles that have an average particle diameter larger than that of the first particles and specifically bind to the target substance, (b) a step for mixing the sample solution, the first particles and the second particles to obtain a mixed solution, and (c) a step for determining at least one of the concentration and the presence or absence of the target substance from polarization anisotropy of the mixed solution obtained in (b)", and thus are classified as invention 1.

(Invention 2) Claims 3-16
    Claim 3 and claim 1 classified as invention 1 pertain to a method for "determining at least one of the concentration and the presence or absence of a target substance in a sample solution" and share the technical feature of having "first particles that specifically bind to the target substance and contain a rare-earth complex and second particles that have an average particle diameter larger than that of the first particles and specifically bind to the target substance".
    However, this technical feature does not make a contribution over the prior art in light of the content disclosed in document 1, and thus cannot be said to be a special technical feature. Moreover, there are no other same or corresponding special technical features among these inventions.
    Furthermore, claims 3-16 are not dependent on claim 1. In addition, claims 3-16 are not substantially identical to or similarly closely related to any of the claims classified as invention 1.
    Accordingly, claims 3-16 cannot be classified as invention 1.
    Claims 3-16 have the special technical feature of a "test kit for determining at least one of the concentration and the presence or absence of a target substance, the kit comprising first particles that specifically bind to the target substance and second particles that have an average particle diameter larger than that of the first particles and specifically bind to the target substance, wherein the first particles contain a rare-earth complex, the first particles have an average particle diameter of 10 nm-1 μm inclusive and the second particles have an average particle diameter of 100 nm-5 μm inclusive", and thus are classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2022/022413**

| Patent document cited in search report | | | Publication date (day/month/year) | Patent family member(s) | | | Publication date (day/month/year) |
|---|---|---|---|---|---|---|---|
| JP | 7-72155 | A | 17 March 1995 | (Family: none) | | | |
| JP | 2000-146975 | A | 26 May 2000 | (Family: none) | | | |
| WO | 2019/132045 | A1 | 04 July 2019 | US<br>paragraphs [0049]-[0051],<br>[0060]<br>EP | 2021/0061820<br><br><br>3733782 | A1<br><br><br>A1 | |
| WO | 2019/208711 | A1 | 31 October 2019 | (Family: none) | | | |
| JP | 2893772 | B2 | 24 May 1999 | (Family: none) | | | |
| JP | 10-512751 | A | 08 December 1998 | WO<br>page 6, line 25 to page 9, line 21<br>EP<br>AU<br>US | 96/22383<br><br><br>805870<br>3140195<br>5641629 | A1<br><br><br>A1<br>A<br>A | |
| US | 2011/0207614 | A1 | 25 August 2011 | (Family: none) | | | |

Form PCT/ISA/210 (patent family annex) (January 2015)

**REFERENCES CITED IN THE DESCRIPTION**

**Patent documents cited in the description**

- JP H0352575 B
- JP 2893772 B
- JP H03103765 A
- JP 2021096211 A